# EUROPEAN PATENT SPECIFICATION

(11) **EP 1 202 951 B1**
(45) Date of publication and mention of the grant of the patent: **22.09.2004**
(21) Application number: 00948161.5
(22) Date of filing: 26.07.2000
(51) Int. Cl.: C07C 69/86

(54) **HYDROGEN BONDED COMPOUNDS**
DURCH WASSERSTOFF GEBUNDENE VERBINDUNGEN
COMPOSES LIES A L'HYDROGENE

(30) Priority: 27.07.1999 GB 9917461
(43) Date of publication of application: 08.05.2002
(73) Proprietor: Smith & Nephew PLC, London WC2N 6LA (GB)
(72) Inventor: GREENER, Bryan, Elvington, York YO41 4BG (GB)
(74) Representative: Draggett, Peter Thornton
(86) International application number: PCT/GB2000/002881
(87) International publication number: WO 2001/007396

(56) References cited:
- BRYAN GREENER: "Melt supramolecular assembly of oligomers with regularly spaced, alternating hydrogen bonding and hydrophobic sites " CHEMICAL COMMUNICATIONS., 1999, pages 2361-2362, XP002150429 ROYAL SOCIETY OF CHEMISTRY., GB ISSN: 1359-7345

## Description

This invention relates to degradable polymer-like materials, in particular to such materials which are biodegradable, to precursors therefor and to artefacts made therefrom such as medical implant devices. More particularly the invention relates to polymer-like materials which can be formed into flexible constructs such as structural blocks, yarns and fibres.

In the conventional understanding of the term polymer, literally, many units, the component sub-units or precursors, eg. monomers or oligomers are bonded together via covalent linkages to form a high molecular weight material. Degradation of the polymer into lower molecular weight species occurs by scission of the covalent bonds binding the sub-units or by scission of a bond within one or more of the sub-units. For materials to biodegrade, the scission mechanism is usually a hydrolytic reaction. For a covalently bound polymer artefact to biodegrade completely, the hydrolysis of the polymer may take several years. Thus such polymers may have limited use in environments where constructs made from such polymers are required to have a temporary existence. Even in those cases where hydrolysis of the covalent bond, for example an anhydride linkage, takes place rapidly there has been no ability to control the precise nature of the degradation product. Thus, in some instances it may be desirable to degrade the polymer to lower molecular weight, non-toxic molecules, such as carbon dioxide and water, but in others it may be desired to form degradation products which are, themselves, beneficial, for example, exhibit a pharmaceutical effect.

Thus, as an object, the present invention seeks to provide a class of materials which are capable of being formed into artefacts and yet can be degraded in a predictable and controlled manner to form predictable fragments.

The materials of the present invention are characterised in that although they are polymer-like, the precursor residues are bonded to each other not by covalent bonds but by hydrogen bonds. Previously, this approach has been successfully applied to produce polymeric species by association of molecules with hydrogen bonding groups at their termini (for example, see R. P. Sijbesma, F. H. Beijer, L. Brunsveld, B. J. B. Folmer, J. H. K. K. Hirschberg, R. F. M. Lange, J. K. L. Lowe and E. W. Meijer, *Science,* **199**.**7**, *278*, 1601 and references cited therein):

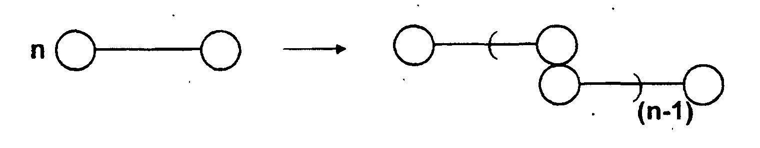

Such materials have been reported to be linear polymers, with each sub-unit associated to its neighbour at one site (which may be comprised of several hydrogen bonding groups). Because every chain is only as strong as its weakest link, researchers have focused on maximising the number of terminal hydrogen bonding groups. In a departure from this approach, we have produced molecules with multiple, regularly spaced hydrogen bonding sites and, in particular, at non-terminal sites, distinct from the prior art in that intermolecular interactions may occur at many sites and in a networked fashion: 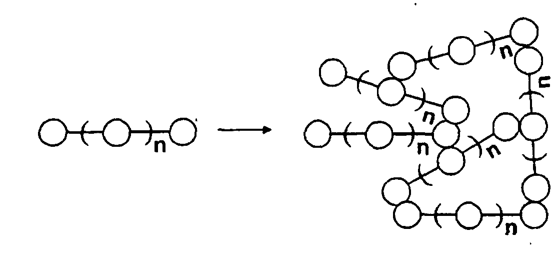 The attachment of molecular components at many interactive sites affords less opportunity for dissociation than those hydrogen bonded molecules or 'assemblies' with only terminal interaction sites reported for prior art species.

In accordance with a first embodiment of the present invention there is provided a supramolecular assembly comprising a plurality of hydrogen bonded molecules, preferably pharmacologically acceptable molecules, each molecule contains multiple site hydrogen bonding groups and wherein at least a proportion of the molecules are bonded to other molecules at sites other than at terminal locations. Aptly the multiple site hydrogen bonding groups are regularly spaced.

In a preferred form of this embodiment the hydrogen bonding sites will be separated by hydrophobic moieties such as a moiety derived from an alkyl diacid

In accordance with a further embodiment of the invention there is provided a compound that is capable of being hydrogen bonded to form a supramolecular assembly and which has the general formula **(I)**:

A-X-(N-**X**)ₙ-A **(I)**

where:
A is an aromatic moeity of the general formula (II):

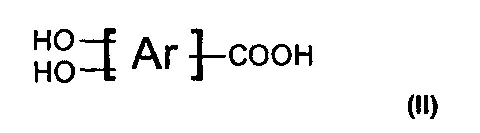

Where **Ar** is an unsubstituted or substituted aromatic nucleus e.g. phenyl or benzyl.
**N** may be the same or different and is a moiety containing at least one hydrogen bond donor and/or acceptor,
*X* is an alkyl diacid, or a functional derivative thereof of the type

HOOC- (CH₂)ₘ-COOH

where m≥2. Aptly, the moiety X may be derived from long chain acids such as dodecandioic-, decanedioic-, or hexanedioic acids or functional derivatives thereof such as dodecadioyl chloride, suberoyl chloride or sebacoyl chloride.
and **n** is an integer having a value of at least one.

In a further embodiment of the invention there is provided a biodegradable composition of matter comprising a super assembly of molecules each having the general formula (I) herein. More preferably, **A** and **N** will contain a plurality of hydrogen bond donor or acceptor sites, typically regularly spaced apart. The A moiety will contain at least four hydrogen bond donor or acceptor sites

The moieties **A** and **N**, containing the donor and/or acceptance sites or groups, may be known per se. Preferred moieties are those that contain hydroxyl and/or carboxyl groups.

Preferred examples of compounds of Formula II are moieties which are capable of site-specific reactivity with the moiety **X**. Such preferred compounds include 2,5- and 2,3-dihydroxybenzoic acids

For example, when **X** is an alkyl diacid chloride, 2,5 dihydoxybenzoic acid will react according to the equation:

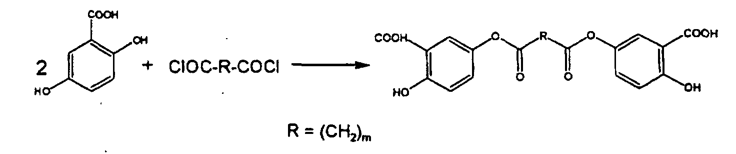

The disposition of the terminal donor and acceptor sites in this compound may be represented thus:

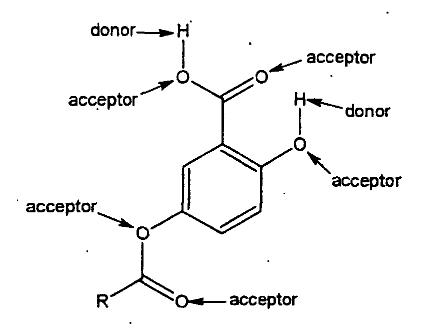

**N** is a moiety containing at least one hydrogen bond acceptance or donation site, aptly two or more hydrogen bond donation or acceptance sites, and may preferably contain at least three donors and/or acceptors. Preferably N is a moiety which comprises both hydrogen bond donating and accepting sites regularly spaced,

The moiety **N** may be the same or different as the moiety **A**. Aptly, where **A** and **N** are different, **A** may be 2,5-dihydroxybenzoic acid and **N** may be 3,5-dihydroxybenzoic acid.

**X** is a difunctional linkage or residue and may be any moiety which does not have an adverse effect on the properties of the donor or acceptor groups. Suitably, **X** may comprise one or more groups which exhibit hydrophobic properties. Aptly, **X** will be a residue which will impart flexibility to aggregates, mixtures or polymers derived from compounds of the invention.

Reactants comprising the precursors of the moieties A and N and **X** are reacted to form covalent linkages between the species. The methods employed to carry out this reaction may by those conventionally employed. For example, **A** or **N** may be connected to **X** *via* an ester linkage by reacting **A** or **N**, comprising of at least one hydroxyl function, with an acid halide of **X** as shown by the following reaction scheme:

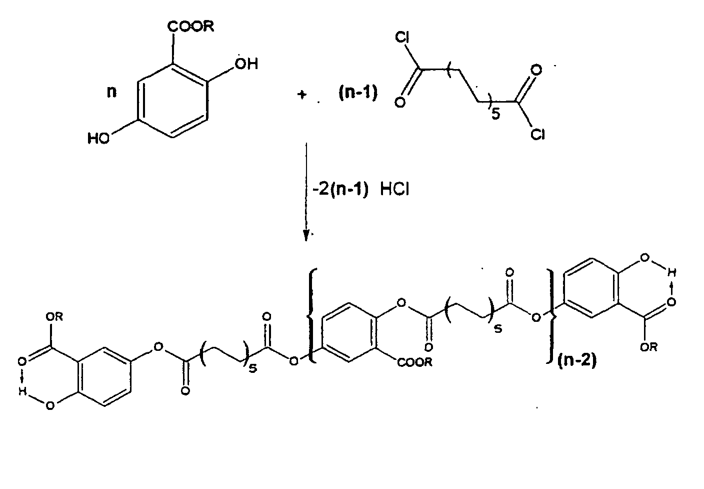

The precursors of the supramolecular assemblies, being compounds and mixtures, as defined above, display aggregative properties in solution and/or in the molten state will henceforth be referred to as 'press-stud oligomers'. Aggregation of press-stud oligomers via the interaction of hydrogen bonding sites A and N allows the formation of supramolecular assemblies (in the form of fibres) when the press stud oligomer mass is melt extruded at elevated temperatures (>50 °C). Fibres so formed are self adherent and flexible immediately after extrusion. Aggregation can be probed by ¹³C NMR spectroscopy and viscometric measurements against reference compounds lacking some/all hydrogen bonding functions.

The fibre forming properties of such aggregates, whilst not fully understood, are believed to be related to the abilility of the oligomers to align themselves under extrusion, as shown:

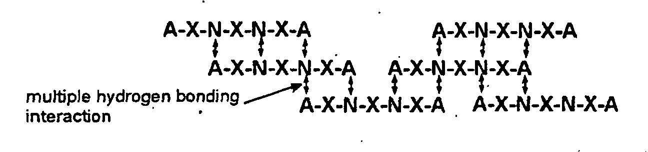

Press-stud oligomers are fibre-forming materials and may be composed of biocompatible and/or therapeutically active compounds (e.g. 2,5-dihydroxybenzoic acid) that are water soluble.

The press-stud oligomers of the present invention may be formed into supramolecular assemblies suitable for use as drug delivery vehicles and adhesives. The press-stud oligomers may be shaped into supramolecular assemblies suitable for medical device applications such as load-bearing fixation plates, screws or tissue anchors. In an alternative use the supramolecular assemblies of the present invention may have uses outside the medical device field, for example as a biodegradable structural packaging material.

Accordingly, the present invention further provides an artefact formed from the biodegradable compositions of matter as described herein.

The invention will now be further described with reference to the accompanying drawings and the following examples, based on:
2,5-dihydroxybenzoic acid (**G**),
dodecanedioyl dichloride (**D**) and
methyl-2,5-dihydroxybenzoate (**MeG**)
all of which were supplied by Aldrich Chemical Co. Ltd and used as supplied.
In the structural formulae given abbreviations given in upper case text (e.g. **G**_{**3**}**D**_{**4**}**)** refer to supramolecular assemblies whereas formulae expressed in lower case text (e.g: **g**_{**3**}**d**_{**4**}**)** refer to the discrete press-stud oligomer form.

IR spectra were collected using a Mattson Galaxy 5020 FTIR spectrometer, samples prepared as cast films from THF for analysis. NMR spectra were collected using a JEOL 270 MHz NMR spectrometer.

Mass spectra were acquired using a Fisons Instruments Autospec Spectrometer. Viscometric measurements were performed using a Carrimed CSL500 constant stress rheometer, using a 4 cm diameter parallel plate and a 200 µm gap. Yields of >85% were recovered from all reactions.

### Liquid Chromatography Conditions

Analyses were carried out using a HP 1100 series chromatograph with a Jupiter C18 5µM 150 × 2mm column. Flow rate 0.2ml/min. HP 1100 DAD 200 to 400nm detector. Samples were dissolved in methanol, injection volume 5 µl. Solvent gradient:

| Time / min. | 0.1% aqueous trifluoroacetic acid / %vol. | 0.1% trifluoroacetic acid in acctonitrile / %vol. |
|---|---|---|
| 0 | 50 | 50 |
| 5 | 50 | 50 |
| 20 | 10 | 90 |
| 40 | 10 | 90 |

### Referring to the accompanying drawings:

- Figure 1.: ¹H NMR (270 MHz, d₈-THF) spectra of oligomers, **G**_{**n**}**D**_{**n-1**} (top) and **MeG**_{**n**}**D**_{**n-1**} (bottom) in the aromatic region.
- Figure 2.: Infra-red absorbance spectra of **G**_{**n**}**D**_{**n-1**} (top) and **MeG**_{**n**}**D**_{**n-1**} (bottom) oligomers.
- Figure 3.: DAD HPLC of **G**_{**3**}**D**_{**2**} showing oligomeric components **g**_{**2**}**d**_{**1**}, **g**_{**3**}**d**_{**2**}, **g**_{**4**}**d**_{**3**} and **g**_{**5**}**d**_{**4**}.
- Figure 4.: details the results of Variable temperature viscometric analysis of **G**_{**n**}**D**_{**n-1**} (top) and **MeG**_{**n**}**D**_{**n-1**} (bottom) oligomers.

### Example 1: Oligomers of the average structure G₃D₂:

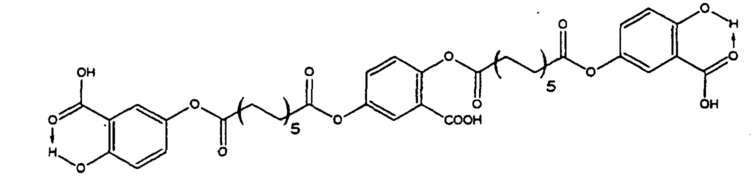

A magnetically stirred melt of 2,5-dihydroxybenzoic acid (4.435 g, 29 mmol) (**G**)and dodecanedioyl dichloride (5.126 g, 19 mmol) (**D**)was heated from ambient temperature to 150°C as rapidly as possible. Following 10 minutes stirring at this temperature, the viscous, transparent melt was poured from the vessel, solidifying to an opaque glass, and desiccated. IR / cm⁻¹: 1132, 1182, 1486, 1698, 1760, 2618, 2854, 2928, 3080. ¹H NMR (270 MHz; d₈-THF): δ11.04 (s (sharp), -OH); δ8.32 (s (broad), -COOH); 2,5-disubstituted **G**: δ7.72 (d, *J* 2.8, Ar-H); δ7.29 (dd, *J* 8.9, 2.8, Ar-H); δ7.08 (d, *J* 8.9, Ar-H); 5-substituted **G**: δ7.54 (d, *J* 2.8, Ar-H); δ7.18 (dd, *J* 8.9, 2.8, Ar-H); δ6.89 (d, *J* 8.9, Ar-H); **D** δ2.51 (t, *J* 7.2, αCH₂); δ1.69 (m, βCH₂); δ1.36 (m, γδεCH₂). Electrospray MS -ve ion: 501.1 **g**_{**2**}**d**_{**1**} 849.2 **g**_{**3**}**d**_{**2**}, 1197.3 **g**_{**4**}**d**_{**3**} (M-H⁺).

### Example 2: Oligomers of the average structure G₄D₃:

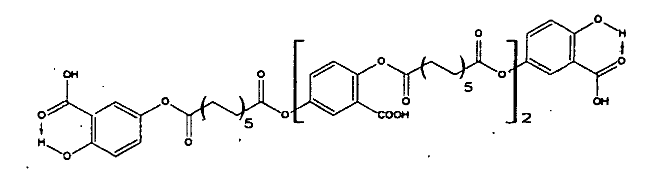

A magnetically stirred melt of 2,5-dihydroxybenzoic acid (4.115 g, 27 mmol) and dodecanedioyl dichloride (5.351 g, 20 mmol) was heated from ambient temperature to 150 °C as rapidly as possible. Following 10 minutes stirring at this temperature, the viscous, transparent melt was poured from the vessel, solidifying to an opaque glass, and desiccated. IR / cm⁻¹: 1132, 1182, 1486, 1698, 1760, 2618, 2854, 2928, 3080. ¹H NMR (270 MHz; d₈-THF): δ11.04 (s (sharp), -OH); δ8.32 (s (broad), -COOH); 2,5-disubstituted **G**: δ7.72 (d, *J* 2.8, Ar-H); δ7.29 (dd, *J* 8.9, 2.8, Ar-H); δ7.08 (d, *J* 8.9, Ar-H); 5-substituted **G**: δ7.54 (d, *J* 2.8, Ar-H); δ7.18 (dd, *J* 8.9, 2.8, Ar-H); δ6.89 (d, *J* 8.9, Ar-H); **D** δ2.51 (t, *J* 7.2, αCH₂); δ1.69 (m, βCH₂); δ1.36 (m, γδεCH₂). Electrospray MS -ve ion: 501.1 **g**_{**2**}**d**_{**1**}, 849.2 **g**_{**3**}**d**_{**2**}, 1197.3 **g**_{**4**}**d**_{**3**}, 1545.4 **g**_{**5**}**d**_{**4**}, 1893.5 **g**_{**6**}**d**_{**5**} (M-H⁺).

### Example 3: Oligomers of the average structure G₅D₄:

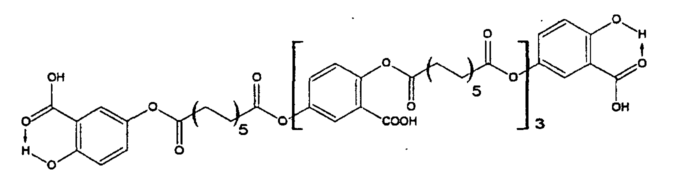

A magnetically stirred melt of 2,5-dihydroxybenzoic acid (3.610 g, 23 mmol) (**G**)and dodecanedioyl dichloride (5.006 g, 19 mmol) (**D**)was heated from ambient temperature to 150°C as rapidly as possible. Following 10 minutes stirring at this temperature, the viscous, transparent melt was poured from the vessel, solidifying to a semi-transparent glass, and desiccated. IR / cm⁻¹: 1132, 1182, 1486, 1698, 1760, 2618, 2854, 2928, 3080. ¹H NMR (270 MHz; d₈-THF): δ11.04 (s (sharp), -OH); δ8.32 (s (broad), -COOH); 2,5-disubstituted **G**: δ7.72 (d, J 2.8, Ar-H); δ7.29 (dd, J 8.9, 2.8, Ar-H); δ7.08 (d, *J* 8.9, Ar-H); 5-substituted **G**: δ7.54 (d, *J* 2.8, Ar-H); δ7.18 (dd, *J* 8.9, 2.8, Ar-H); δ6.89 (d, *J* 8.9, Ar-H); **D** δ2.51 (t, *J* 7.2, αCH₂); δ1.69 (m, βCH₂); δ1.36 (m, γδεCH₂).

### Example 4: Oligomers of the average structure G₆D₅:

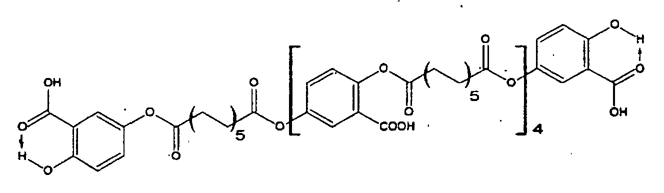

A magnetically stirred melt of 2,5-dihydroxybenzoic acid (3.481 g, 23 mmol) **(G)**and dodecanedioyl dichloride (5.009 g, 19 mmol) **(D)** was heated from ambient temperature to 150°C as rapidly as possible. Following 10 minutes stirring at this temperature, the viscous, transparent melt was poured from the vessel, solidifying to a semi-transparent glass, and desiccated. IR / cm⁻¹: 1132, 1182, 1486, 1698, 1760, 2618, 2854, 2928, 3080. ¹H NMR (270 MHz; d₈-THF): δ11.04 (s (sharp), -OH); δ8.32 (s (broad), -COOH); 2,5-disubstituted **G**: δ7.72 (d, *J* 2.8, Ar-H); δ7.29 (dd, *J* 8.9, 2.8, Ar-H); δ7.08 (d, *J* 8.9, Ar-H); 5-substituted **G**: δ7.54 (d, *J* 2.8, Ar-H); δ7.18 (dd, *J* 8.9, 2.8, Ar-H); δ6.89 (d, *J* 8.9, Ar-H); **D** δ2.51 (t, *J* 7.2, αCH₂); δ1.69 (m, βCH₂); δ1.36 (m, γδεCH₂).

### Example 5: Oligomer of the structure g₃d₂:

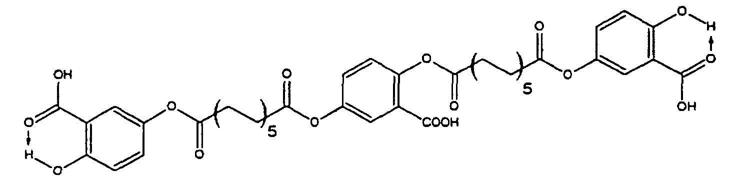

The oligomer of average structure **G**_{**3**}**D**_{**2**} (example 1) was separated by preparative-scale LC into its constituent oligomeric components, resulting in the isolation of **g**_{**3**}**d**_{**2**}. IR / cm⁻¹: 1132, 1182, 1486, 1698, 1760, 2618, 2854, 2928, 3080. ¹H NMR (270 MHz; d₈-THF): δ11.04 (s (sharp), -OH); δ8.32 (s (broad), -COOH); 2,5-disubstituted G: δ7.72 (d, *J* 2.8, Ar-H); δ7.29 (dd, *J* 8.9, 2.8, Ar-H); δ7.08 (d, *J* 8.9, Ar-H); 5-substituted **G**: δ7.54 (d, *J* 2.8, Ar-H); δ7.18 (dd, *J* 8.9, 2.8, Ar-H); δ6.89 (d, *J* 8.9, Ar-H); **D** δ2.51 (t, *J* 7.2, αCH₂); δ1.69 (m, βCH₂); δ1.36 (m; γδεCH₂). Electrospray MS -ve ion: 849.2 (M-H⁺).

### Example 6: Oligomer of the structure g₄d₃:

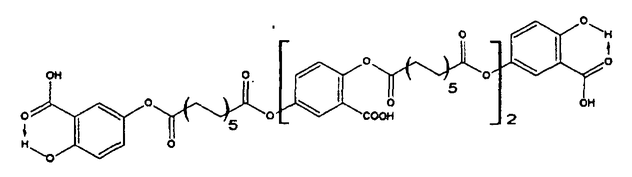

The oligomer of average structure **G**_{**3**}**D**_{**2**} (example 1) was separated by preparative-scale LC into its constituent oligomeric components, resulting in the isolation of **g**_{**4**}**d**_{**3**}. IR / cm⁻¹: 1132, 1182, 1486, 1698, 1760, 2618, 2854, 2928, 3080. ¹H NMR (270 MHz; d₈-THF): δ11.04 (s (sharp), -OH); δ8.32 (s (broad), -COOH); 2,5-disubstituted **G**: δ7.72 (d, *J* 2.8, Ar-H); δ7.29 (dd, *J* 8.9, 2.8, Ar-H); δ7.08 (d, *J* 8.9, Ar-H); 5-substituted **G**: δ7.54 (d, J 2.8, Ar-H); δ7.18 (dd, J 8.9, 2.8, Ar-H); δ6.89 (d, *J* 8.9, Ar-H); **D** δ2.51 (t, J 7.2, αCH₂); δ1.69 (m, βCH₂); δ1.36 (m, γδεCH₂). Electrospray MS -ve ion: 1197.3 (M-H⁺).

### Example 7: Oligomer of the average structure G₃D₃

A magnetically stirred melt of 2,5-dihydroxybenzoic acid (7.518 g, 49 mmol) and dodecanedioyl chloride (13.034 g, 49 mmol) was heated to 150 °C. Following 10 minutes of heating at this temperature, the transparent viscous melt was cooled to room temperature and desiccated.

### Mechanical Properties

The mechanical properties of some of the supramolecular assemblies of the present invention are given below.

Aluminium studs were provided with a raised circular portion 5mm in diameter. A melt of the oligomers listed in Table 1 were coated onto the raised circular portions and the coated circular portions two studs were brought and held together under hand pressure until the melt had cooled and solidified. For comparative purposes a pair of aluminium studs were joined together with a conventional cyanoacrylate adhesive in the same manner as the supra molecular assemblies of the invention

Each stud was held in the jaws of a Nene MC 30000 tensile testing machine and testing was carried out a speed of 5mm min⁻¹.

**Table 1**

| Example | Oligomer | Load to break / N | Breaking strength / MPa |
|---|---|---|---|
| | **G**_{**2**}**D**_{**1**} | 50 | 1.8 |
| **1** | **G**_{**3**}**D**_{**2**} | 413 | 15.1 |
| **2** | **G**_{**4**}**D**_{**3**} | 222 | 8.1 |
| **3** | **G**_{**5**}**D**_{**4**} | 105 | 3.8 |
| **4** | **G**_{**6**}**D**_{**5**} | 202 | 7.4 |
| | Cyanoacrylate | 193 | 7.1 |

For physical comparison with examples 1-4, equivalent oligomers were prepared using methyl-2,5-dihydroxybenzoate (**MeG**) in place of 2,5-dihydroxybenzoic acid:

### COMPARATIVE EXAMPLES

### (i) - Oligomers of average structure MeG₃D₂

A magnetically stirred melt of methyl-2,5-dihydroxybenzoate (2.461 g, 15 mmol) and dodecanedioyl dichloride (2.607 g, 10 mmol) was heated from ambient temperature to 150 °C as rapidly as possible. Following 10 minutes stirring at this temperature, the viscous, transparent melt was poured from the vessel, solidifying to an opaque glass, and desiccated. IR / cm⁻¹: 1129, 1212, 1486, 1682, 1731, 1761, 2854, 2928. ¹H NMR (270 MHz; d₈-THF): δ10.60 (2H, s (sharp), -OH); 2,5-disubstituted **MeG**: δ7.69 (d, *J* 3.0, Ar-H); δ7.31 (dd, *J* 8.7, 2.8, Ar-H); δ7.11 (d, *J* 8.7, Ar-H); δ3.78 (s, CH₃); 5-substituted **MeG**: δ7.52 (d, *J* 3.0, Ar-H); δ7.21 (dd, *J* 8.7, 3.0, Ar-H); δ6.93 (d, *J* 8.7, Ar-H); δ3.91 (s, CH₃); **D** δ2.51 (t, *J* 7.2, αCH₂); δ1.69 (m, βCH₂); δ1.36 (m, γδεCH₂).

### (ii) - Oligomers of average structure MeG₄D₃

A magnetically stirred melt of methyl-2,5-dihydroxybenzoate (2.426 g, 14 mmol) and dodecanedioyl dichloride (2.892 g, 11 mmol) was heated from ambient temperature to 150°C as rapidly as possible. Following 10 minutes stirring at this temperature, the viscous, transparent melt was poured from the vessel, solidifying to an opaque glass, and desiccated. IR / cm⁻¹: 1129, 1212, 1486, 1682, 1731, 1761, 2854, 2928. ¹H NMR (270 MHz; d₈-THF): δ10.60 (2H, s (sharp), -OH); 2,5-disubstituted **MeG**: δ7.69 (d, *J* 3.0, Ar-H); δ7.31 (dd, *J* 8.7, 2.8, Ar-H); δ7.11 (d, *J* 8.7, Ar-H); δ3.78 (s, CH₃); 5-substituted **MeG**: δ7.52 (d, J 3.0, Ar-H); δ7.21 (dd, *J* 8.7, 3.0, Ar-H); δ6.93 (d, *J* 8.7, Ar-H); δ3.91 (s, CH₃); **D** δ2.51 (t, *J* 7.2, αCH₂); δ1.69 (m, βCH₂); δ1.36 (m, γδεCH₂).

### (iii) - Oligomers of average structure MeG₅D₄

A magnetically stirred melt of methyl-2,5-dihydroxybenzoate (3.934 g, 23 mmol) and dodecanedioyl dichloride (5.013 g, 19 mmol) was heated from ambient temperature to 150°C as rapidly as possible. Following 10 minutes stirring at this temperature, the viscous, transparent melt was poured from the vessel, solidifying to a semi-transparent glass, and desiccated. IR / cm⁻¹: 1129, 1212, 1486, 1682, 1731, 1761, 2854, 2928. ¹H NMR (270 MHz; d₈-THF): δ10.60 (2H, s (sharp), -OH); 2,5-disubstituted **MeG**: δ7.69 (d, *J* 3.0, Ar-H); δ7.31 (dd, *J* 8.7, 2.8, Ar-H); δ7.11 (d, *J* 8.7, Ar-H); δ3.78 (s, CH₃); 5-substituted **MeG**: δ7.52 (d, *J* 3.0, Ar-H); δ7.21 (dd, *J* 8.7, 3.0, Ar-H); δ6.93 (d, *J* 8.7, Ar-H); δ3.91 (s, CH₃); **D** δ2.51 (t, *J* 7.2, αCH₂); δ1.69 (m, βCH₂); δ1.36 (m, γδεCH₂).

### (iv) - Oligomers of average structure MeG₆D₅

A magnetically stirred melt of methyl-2,5-dihydroxybenzoate (3.778 g, 23 mmol) and dodecanedioyl dichloride (5.016 g, 19 mmol) was heated from ambient temperature to 150°C as rapidly as possible. Following 10 minutes stirring at this temperature, the viscous, transparent melt was poured from the vessel, solidifying to a semi-transparent glass, and desiccated. IR / cm⁻¹: 1129, 1212, 1486, 1682, 1731, 1761, 2854, 2928. ¹H NMR (270 MHz; d₈-THF): δ10.60 (2H, s (sharp), -OH); 2,5-disubstituted **MeG**: δ7.69 (d, *J* 3.0, Ar-H); δ7.31 (dd, *J* 8.7, 2.8, Ar-H); δ7.11 (d, *J* 8.7, Ar-H); δ3.78 (s, CH₃); 5-substituted **MeG**: δ7.52 (d, *J* 3.0, Ar-H); δ7.21 (dd, *J* 8.7, 3.0, Ar-H); δ6.93 (d, *J* 8.7, Ar-H); δ3.91 (s, CH₃); **D** δ2.51 (t, *J* 7.2, αCH₂); δ1.69 (m, βCH₂); δ1.36 (m, γδεCH₂).

The MeG-oligomers so produced differed from the examples of the invention in that the potential for intermolecular acid hydrogen bonding had been removed.

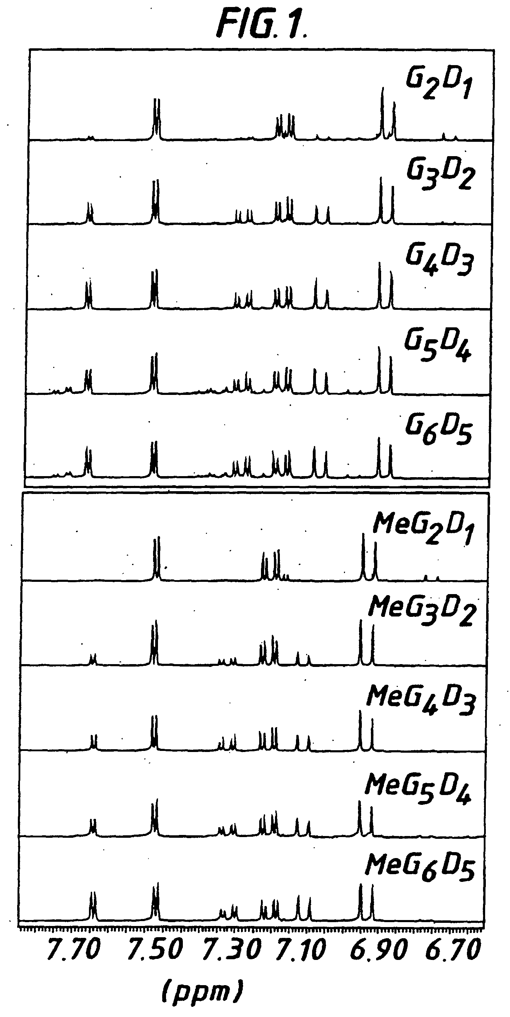

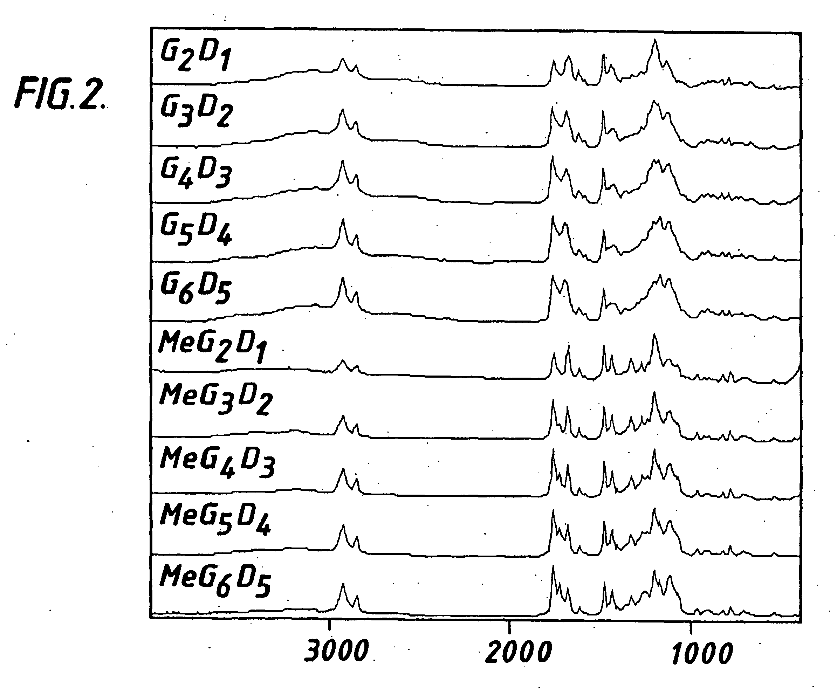

Structural and oligomeric homology between the **G**-based and **MeG**-based oligomers was confirmed by ¹H NMR spectroscopy, as shown in Figure 1. The presence of acidic hydrogen bonding functionality in the **G**-based oligomers and the absence of such functionality in **MeG**-based oligomers manifested itself when the IR spectra of the two series were compiled and compared, as seen in Figure 2. The absorbance band-broadening observed in the carbonyl region (ca. 1700 cm⁻¹) for **G**-based oligomers is indicative of several hydrogen bonding environments, in comparison with relatively sharp absorbances in corresponding **MeG**-based oligomers,

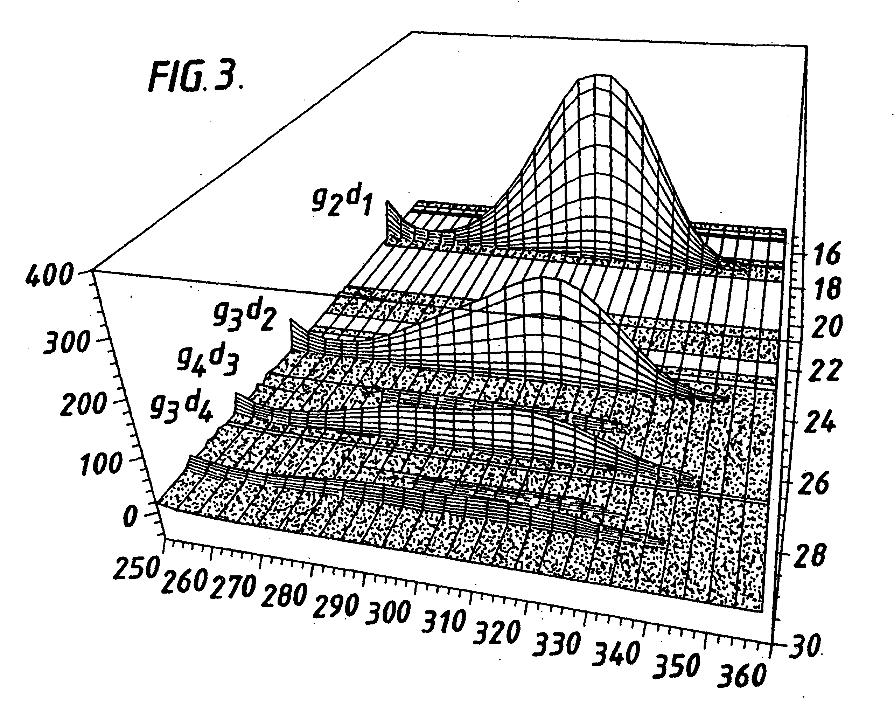

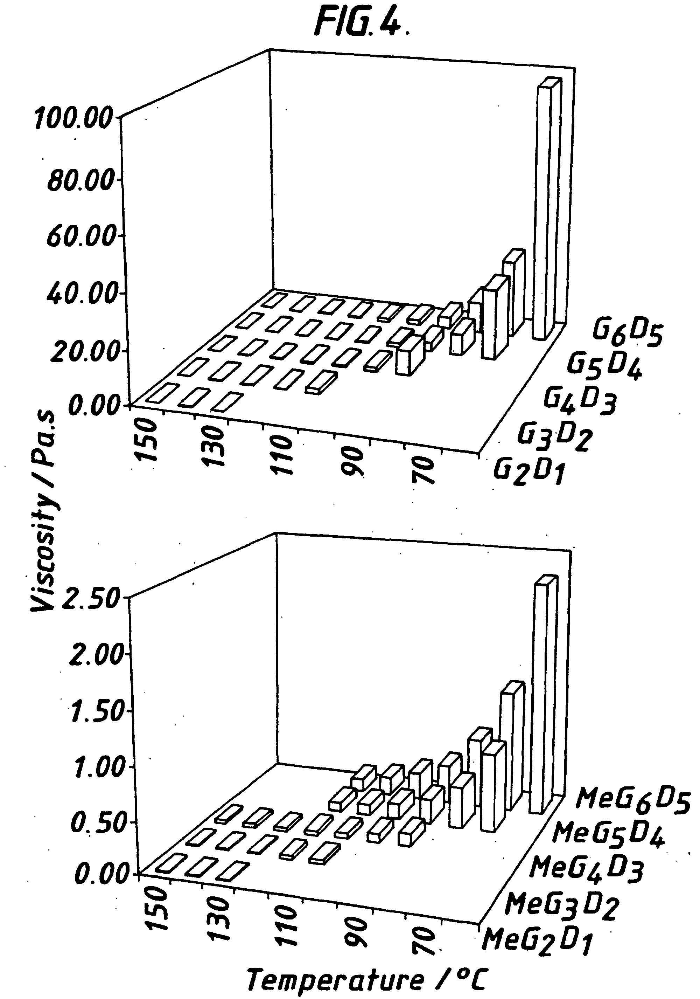

The oligomeric distribution for examples of average structure was determined by liquid chromatography with a UV-vis diode array detector. The results shown in Figure 3 illustrate the distribution of oligomers in the Supramolecular Assembly described in Example 1. The proposed physical effect of multiple-site intermolecular hydrogen bonding interactions was confirmed by variable temperature viscometric study of **G**-based and **MeG**-based oligomers, as shown in Figure 4. The viscosities for **G**-based oligomers were consistently greater than those observed for **MeG**-based oligomers by ca. 40-fold. It can also be seen that, in general, viscosities increased, throughout the temperature range observed, as the average oligomeric length increased. Viscosities increased with a greater rate towards solidification as the average oligomeric length increased. These observations are in accordance with an increasing number of intermolecular hydrogen bonding interactions and entanglements.

All **G**_{**n**}**D**_{**n-1**} oligomers formed fibres from the molten state that became brittle after several minutes at room temperature; **MeG**_{**n**}**D**_{**n-1**} oligomers were non-fibre-forming. All **G**_{**n**}**D**_{**n-1**} and **MeG**_{**n**}**D**_{**n-1**} oligomers cooled to semi-transparent glasses.

## Claims

1. A compound that is capable of being hydrogen bonded to form a supramolecular assembly, said compound having the general formula **(I)**:
A-X-(N-**X**)ₙ-A **(I)** (I)
where:
**A** may be the same or different and is an aromatic moiety of the general formula (II): 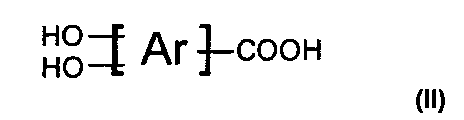
Where **Ar** is an unsubstituted or substituted aromatic nucleus.
**N** may be the same or different and is a moiety containing at least one hydrogen bond donor and/or acceptor,
**X** may be the same or different and is a residue of an alkyl diacid of the general formula:
HOOC-(CH₂)ₘ-COOH
or a functional derivative thereof, wherein m is an integer having a value of at least 2,
and **n** is an integer having a value of at least one.

2. A compound as claimed in claim 1 wherein **Ar** is phenyl or benzyl

3. A compound as claimed in either claim 1 or claim 2 wherein the compound of Formula (II) is 2,5- dihydroxybenzoic acid or 2,3-dihydroxybenzoic acid

4. A compound as claimed in any of claims 1 to 3 wherein **N** is a moiety containing at least three hydrogen bond acceptance or donation sites.

5. A compound as claimed in any one of claims 1 to 4 wherein **X** is derived from dodecanedioic-, decanedioic-, octanedioic- or hexanedioic acids or an acid chloride thereof

6. A supramolecular assembly comprising a plurality of hydrogen bonded molecules comprising the aggregation of compounds of the general formula (I) as defined in any one of claims 1 to 5.

7. An artefact manufactured from an assembly as claimed in claim 6 or from a compound as claimed in any one of claims 1 to 5.

8. A process for the manufacture of an artefact as defined in claim 7 comprising subjecting said assembly to melt extrusion at a temperature >50°C to form self-adherent fibres.

## Patentansprüche

1. Eine Verbindung, die durch eine Wasserstoffbrücke verbunden werden kann, um ein Assoziat zu bilden, wobei die Verbindung von folgender allgemeinen Formel (I) ist:
A-X-(N-X)ₙ-A (I)
wobei:
A gleich oder unterschiedlich sein kann und ein aromatischer Teil der allgemeinen Formel (II) ist: 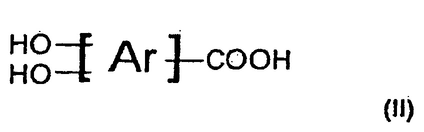
Wobei Ar ein unsubstituierter oder substituierter aromatischer Kern ist,
N gleich oder unterschiedlich sein kann und ein Teil ist, der mindestens einen Wasserstoffbrückenbindungs-Donator und/oder - akzeptor enthalten kann,
X gleich oder unterschiedlich sein kann und ein Rest einer zweiwertigen Alkylsäure der folgenden allgemeinen Formel ist:
HOOC-(CH₂)ₘ-COOH
oder ein funktionelles Derivat davon, wobei m eine ganze Zahl mit einem Wert von mindestens 2 ist,
und n eine ganze Zahl mit einem Wert von mindestens eins ist.

2. Verbindung gemäß Anspruch 1, wobei, wobei Ar Phenyl oder Benzyl ist.

3. Verbindung gemäß Anspruch 1 oder Anspruch 2, wobei die Verbindung der Formel (II) 2,5-Dihydroxybenzoesäure oder 2,3-Dihydroxybenzoesäure ist.

4. Verbindung gemäß einem der Ansprüche 1 bis 3, wobei N ein Teil, der mindestens drei Wasserstoffbrückenbindungs-Aufnahmeorte oder -Abgabeorte enthält, ist.

5. Verbindung gemäß einem der Ansprüche 1 bis 4, wobei X von Dodecandi-, Decandi-, Octandi- oder Hexandisäuren oder einem Säurechlorid davon hergeleitet ist.

6. Ein Assoziat, das eine Vielzahl von durch Wasserstoffbrücken verbundene Moleküle beinhaltet, welche die Aggregation von Verbindungen der allgemeinen Formel (I), wie in einem der Ansprüche 1 bis 5 definiert, beinhalten.

7. Ein Artefakt, das aus einem Assoziat, wie in Anspruch 6 beansprucht, oder aus einer Verbindung, wie in einem der Ansprüche 1 bis 5 beansprucht, hergestellt wurde.

8. Ein Verfahren zur Herstellung eines wie in Anspruch 7 definierten Artefakts, das beinhaltet, dass das Assoziat bei einer Temperatur von >50 °C Schmelzspinnen unterzogen wird, um selbstklebende Fasern zu bilden.

## Revendications

1. Un composé qui est capable d'être lié à l'hydrogène pour former un assemblage supramoléculaire, ledit composé ayant la formule générale (I) :
A-X-(N-X)ₙ-A (I)
où :
A peut être identique ou différent et est un fragment aromatique de la formule générale (II) : 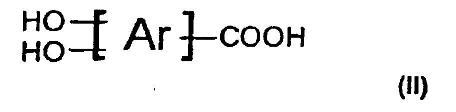
où Ar est un noyau aromatique non substitué ou substitué.
N peut être identique ou différent et est un fragment contenant au moins un donneur et/ou un accepteur de liaison hydrogène,
X peut être identique ou différent et est un résidu d'un diacide d'alkyle de la formule générale :
HOOC-(CH₂)ₘ-COOH
ou un dérivé fonctionnel de celui-ci, dans lequel m est un entier ayant une valeur d'au moins 2,
et n est un entier ayant une valeur d'au moins un.

2. Un composé tel que revendiqué dans la revendication 1 dans lequel Ar est phényl ou benzyl.

3. Un composé tel que revendiqué soit dans la revendication 1, soit dans la revendication 2, dans lequel le composé de la formule (II) est de l'acide 2,5-dihydroxybenzoïque ou de l'acide 2,3-dihydroxybenzoïque.

4. Un composé tel que revendiqué dans n'importe lesquelles des revendications 1 à 3 dans lequel N est un fragment contenant au moins trois sites d'acceptation ou de don de liaison hydrogène.

5. Un composé tel que revendiqué dans n'importe laquelle des revendications 1 à 4 dans lequel X est dérivé d'acides dodécanedioïque, sébacique, subérique ou hexanedioïque ou d'un chlorure d'acide de ceux-ci.

6. Un assemblage supramoléculaire comportant une pluralité de molécules liées à l'hydrogène comportant l'agrégation de composés de la formule générale (I) tels que définis dans n'importe laquelle des revendications 1 à 5.

7. Un article fabriqué à partir d'un assemblage tel que revendiqué dans la revendication 6 ou à partir d'un composé tel que revendiqué dans n'importe laquelle des revendications 1 à 5.

8. Un procédé pour la fabrication d'un article tel que défini dans la revendication 7 comportant le fait de soumettre ledit assemblage à une extrusion de matière fondue à une température >50 °C pour former des fibres auto-adhésives.
